# EUROPEAN PATENT APPLICATION

(11) **EP 4 821 490 A1**
(43) Date of publication of application: **14.10.2026**
(21) Application number: 25382382.7
(22) Date of filing: 11.04.2025
(51) Int. Cl.: A61K 9/51

(54) **CHITOSAN-BASED NANOCAPSULE SYSTEM FOR USE IN THE PROPHYLACTIC TREATMENT OF AN INFECTION CAUSED BY A RESPIRATORY PATHOGEN**

(71) Applicant: Smart Vitamins SL, 15782 Santiago de Compostela A Coruña (ES); Universidade de Santiago de Compostela, 15782 Santiago de Compostela (ES)
(72) Inventor: ALONSO FERNÁNDEZ, María José, 15782 Santiago de Compostela (ES); BERMÚDEZ DE CASTRO FRANCO, Alfredo, 15782 Santiago de Compostela (ES); RIOBOO VIDAL, Alicia, 15782 Santiago de Compostela (ES); TORRES LÓPEZ, María Dolores Ramona, 15782 Santiago de Compostela (ES)
(74) Representative: Hoffmann Eitle

(57) **Abstract**

The present invention refers to the medical field. Particularly, it refers to a chitosan-based nanocapsule system for use in the prophylactic treatment of an infection caused by a respiratory pathogen, preferably the influenza virus, wherein the nanocapsule system is administered intranasally.

## Description

### TECHNICAL FIELD

The present invention refers to the medical field. Particularly, it refers to a chitosan-based nanocapsule system for use in the prophylactic treatment of an infection caused by a respiratory pathogen, preferably the influenza virus, wherein the nanocapsule system is administered intranasally.

### BACKGROUND ART

Infectious respiratory diseases are illnesses caused by pathogenic microorganisms - such as viruses, bacteria, or fungi - that invade the respiratory system, leading to infections in structures like the nasal passages, throat, airways, and lungs. They represent a significant global health challenge, with influenza being a major contributor to morbidity and mortality worldwide. The disease burden of these diseases extends beyond respiratory illness, impacting cardiovascular health, exacerbating chronic conditions, and increasing susceptibility to secondary bacterial infections. According to estimates, influenza-associated respiratory deaths range from 290,000 to 650,000 annually, highlighting its substantial impact on public health.

Given the considerable health and economic consequences of infectious respiratory diseases, particularly influenza, there is a pressing need to develop effective prevention strategies. Vaccination remains the primary and most effective method of influenza prevention, offering significant protection against the virus and its complications. However, the medical community recognizes an ongoing need for improved prophylactic treatments that can enhance existing preventive measures. Such advancements could be particularly beneficial for high-risk individuals, healthcare workers, and in scenarios where additional protection is required.

The development of novel and more effective preventive strategies for infectious respiratory diseases, especially influenza, could significantly reduce the burden on healthcare systems, decrease mortality rates, and improve overall public health outcomes.

In this context, the present invention provides a nanocapsule system designed as an intranasal prophylactic treatment against respiratory pathogens, particularly against the influenza virus.

### SUMMARY OF THE INVENTION

As explained in the background art, there is a need for effective preventive strategies for infectious respiratory diseases, particularly for respiratory diseases caused by the influenza virus.

The inventors have developed a chitosan-based nanocapsule that serves as an intranasal prophylactic treatment against respiratory pathogens, particularly the influenza virus. This nanocapsule consists of a chitosan-based shell encapsulating an oily core, which is stabilized through the use of an oil-soluble surfactant **(Table 4).**

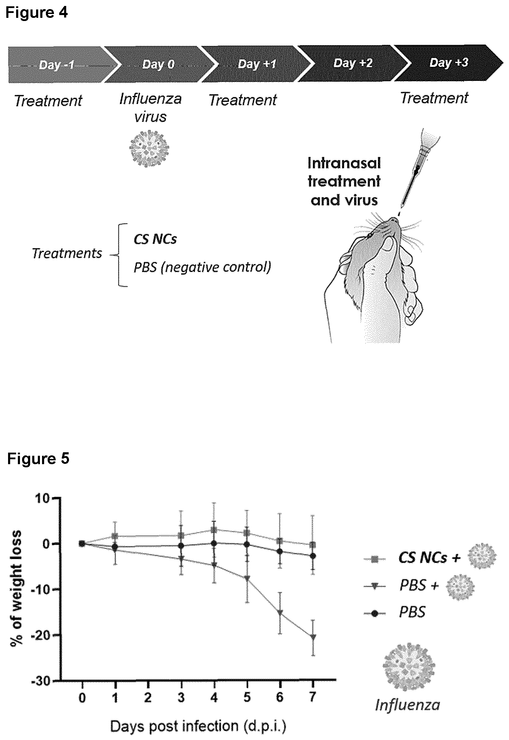

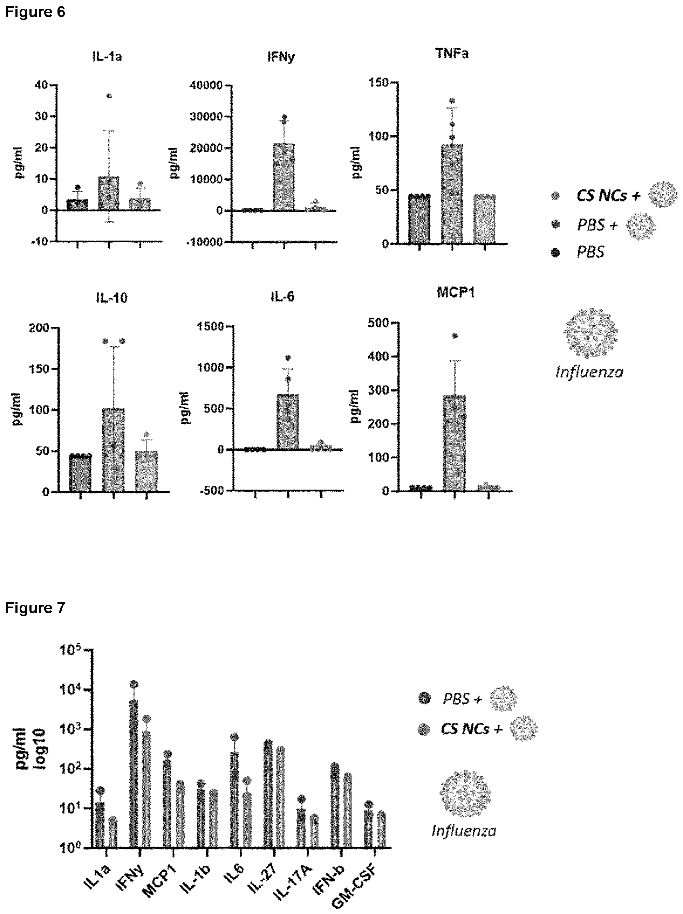

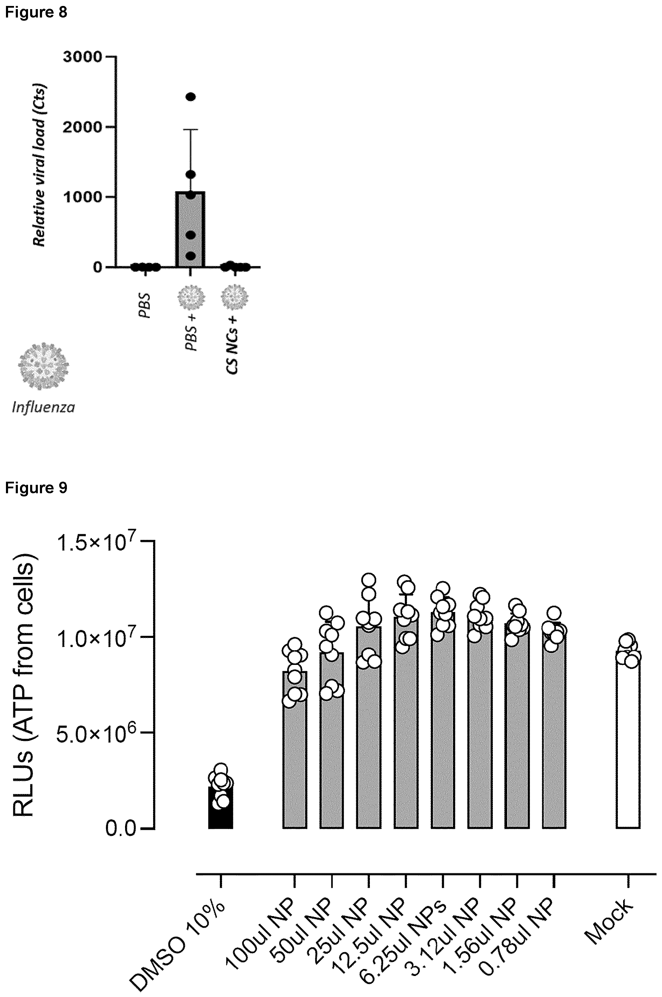

The results provided in this application demonstrate that intranasal administration of these nanocapsules prevent the respiratory pathogen from causing a symptomatic infection, more precisely, from penetrating the nasal epithelium, leading to a complete prevention of respiratory infection caused by influenza. This is particularly supported by an *in vivo* experiment performed on mice exposed to the H1N1 PR8 influenza virus. Mice that received intranasal administration of the chitosan-based nanocapsules on days -1, +1, and +3 relative to viral exposure **(****Figure 4****)** exhibited the following outcomes:
- Prevention of weight loss associated with influenza infection: Mice treated with the nanocapsules maintained a body weight comparable to unexposed healthy mice **(****Figure 5****).**
- Prevention of inflammatory cytokine release in the lungs and bloodstream: Levels of inflammatory cytokines in the lungs **(****Figure 6****)** of treated mice were comparable to those observed in unexposed mice.
- Reduction of inflammatory cytokines in the bloodstream: Levels of inflammatory cytokines in the blood of treated mice were lower than those observed for untreated mice **(****Figure 7****).**
- Complete blockage of influenza virus entry into the nasal mucosa: Mice treated with the nanocapsules prior to viral exposure showed lung viral loads comparable to unexposed mice, indicating the nanocapsules effectively prevented viral particles from entering the nasal epithelium **(****Figure 8****).**

This data indicates that intranasal administration of the nanocapsules completely prevents influenza infection by forming a protective barrier that blocks viral entry at the primary site of infection (i.e. the nasal mucosa).

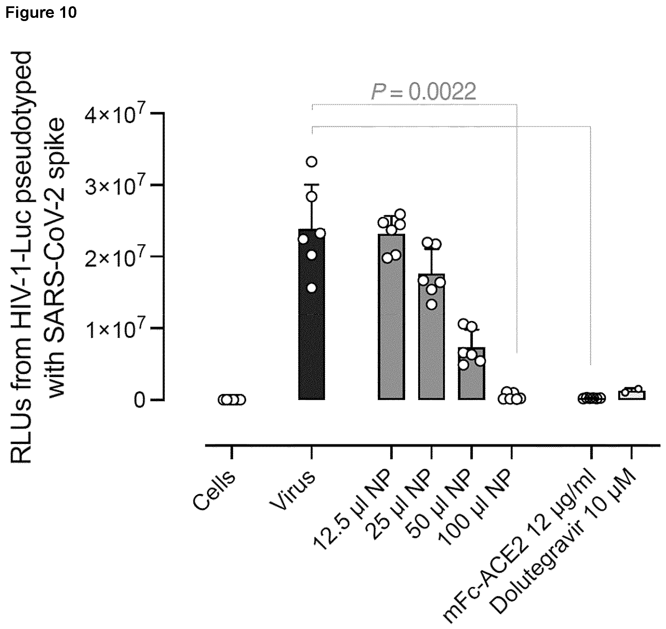

In line with these results, the chitosan nanocapsules inhibited the fusion of a SARS-CoV-2 Spike-containing pseudovirus in ACE2 expressing 293T cells as effectively as recombinant murine Fc-ACE2 protein and HIV-1 integration inhibitors like dolutegravir, both of which have shown potential effects against SARS-CoV-2 **(****Figure 10****).** These findings further support the notion that the chitosan nanocapsules form a protective barrier that inhibits viral entry into cells and suggest that the protective effect observed against the influenza virus is also applicable to other viral respiratory infections, such as SARS-CoV-2.

Unlike previously disclosed chitosan-based nanocapsules, these nanocapsules do not act as adjuvants, modulators of active ingredient activity, or delivery vehicles for other active compounds. Notably, these nanocapsules are not loaded with additional active ingredients. Instead, they possess an inherent prophylactic effect resulting from the combination of the nanocapsules' components. This effect was demonstrated by the 100% prevention rate in animal models treated intranasally on days -1, +1, and +3 relative to viral exposure **(****Figure 8****).**

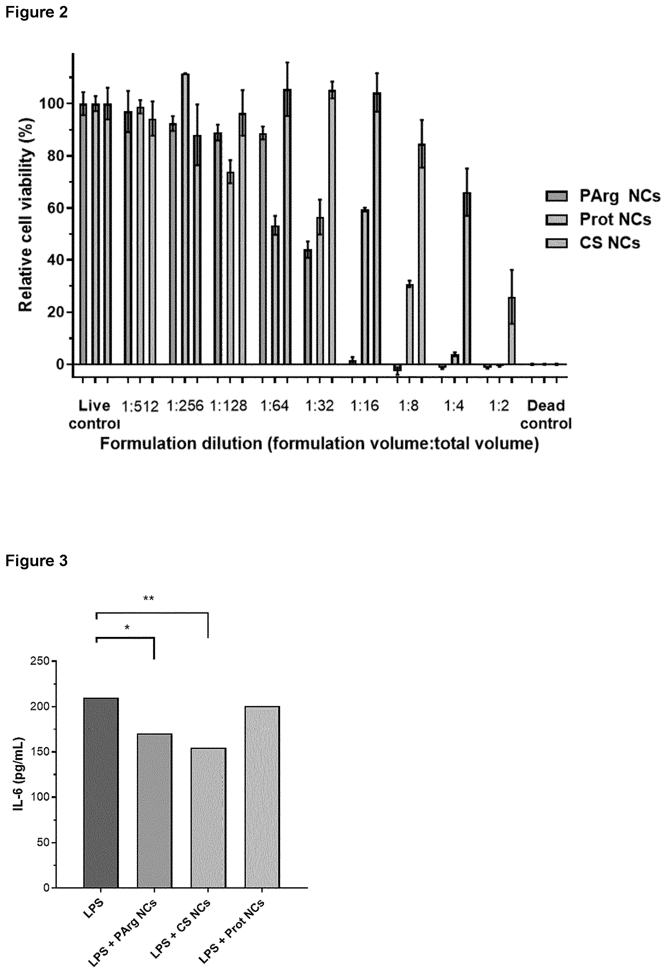

Additionally, toxicity studies **(****Figure 2****)** confirmed that the chitosan-based nanocapsule formulation is non-toxic, providing significantly higher cell viability compared to alternative formulations. Even at high concentrations (dilution 1:4), the nanocapsules maintained cell viability above 50%, supporting their safety for nasal administration.

In conclusion, these nanocapsules offer a threefold advantage: they act as a protective barrier against viral entry, exhibit an intrinsic prophylactic effect, and ensure low toxicity - making them a promising innovation for preventing influenza and other infectious respiratory diseases. Remarkably, the outstanding efficacy of these nanocapsules is achieved with extremely low amounts of chitosan, which brings several advantages. Using such minimal concentrations ensures exceptionally low toxicity while maintaining a high level of prophylactic effectiveness, as demonstrated by the complete prevention of influenza infection in treated mice. Additionally, reducing the quantity of chitosan required for the treatment enhances patient comfort, as lower doses typically result in a more tolerable administration experience. This aspect is crucial for improving patient adherence to the prophylactic regimen, ultimately increasing the overall success of the preventive strategy.

### The invention provides:

A nanocapsule system, which comprises or consists of:
   a) a surface layer comprising chitosan
   b) an oily core, and
   c) a surfactant,
for use in the prophylactic treatment of an infection caused by a respiratory pathogen in a subject in need thereof, wherein the nanocapsule is administered to the subject intranasally.

In a preferred embodiment, optionally in combination with any of the previous or subsequent embodiments, the prophylactic treatment comprises or consists of preventing the respiratory pathogen from causing a symptomatic infection.

In another preferred embodiment, optionally in combination with any of the previous or subsequent embodiments, the prophylactic treatment comprises or consists of preventing the respiratory pathogen from causing a symptomatic infection by preventing the respiratory pathogen from entering the nasal epithelium by forming a protective barrier.

In another preferred embodiment, optionally in combination with any of the previous or subsequent embodiments, the prophylactic treatment comprises or consists of preventing the respiratory pathogen from entering the nasal epithelium by forming a protective barrier.

In another preferred embodiment, optionally in combination with any of the previous or subsequent embodiments, the nanocapsule system is for the specific use in the prophylactic treatment of an infection caused by a respiratory virus. Preferably, the respiratory virus is selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses, bocavirus, and enteroviruses. More preferably, the respiratory virus is the influenza virus. Preferably, the influenza virus is an influenza A virus, preferably H1N1 influenza.

In another preferred embodiment, optionally in combination with any of the previous or subsequent embodiments, the nanocapsule system is for the specific use in the prophylactic treatment of an infection caused by a respiratory virus is selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses, bocavirus, and enteroviruses. More preferably, the respiratory virus is the influenza virus.

In another preferred embodiment, optionally in combination with any of the previous or subsequent embodiments, the nanocapsule system is for the specific use in the prophylactic treatment of an infection caused by the influenza virus. Preferably, the influenza virus is an influenza A virus, preferably H1N1 influenza.

In another preferred embodiment, optionally in combination with any of the previous or subsequent embodiments, the nanocapsule system is for the specific use in the prophylactic treatment of an infection caused by an influenza A virus, preferably H1N1 influenza.

In another preferred embodiment, optionally in combination with any of the subsequent embodiments, the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof.

In another preferred embodiment, optionally in combination with any of the previous or subsequent embodiments, the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, and the prophylactic treatment comprises or consists of preventing the respiratory pathogen from causing a symptomatic infection.

In another preferred embodiment, optionally in combination with any of the previous or subsequent embodiments, the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, the nanocapsule system is for the specific use in the prophylactic treatment of an infection caused by the influenza virus, wherein the prophylactic treatment comprises or consists of preventing the respiratory pathogen from causing a symptomatic infection. Preferably, the influenza virus is an influenza A virus, preferably H1N1 influenza.

In another preferred embodiment, optionally in combination with any of the previous or subsequent embodiments, the oil consists of 90% w/w of triglycerides with three fatty acids having an aliphatic tail of 6 to 12 carbon atoms.

In another preferred embodiment, optionally in combination with any of the previous or subsequent embodiments, the oil consists of at least of 90% w/w of a mixture of caprylic (C8) and capric (C10) triglycerides.

In another preferred embodiment, optionally in combination with any of the previous or subsequent embodiments, the oil consists of at least 90% w/w of a mixture of caprylic (C8) and capric (C10) triglycerides in a w/w ratio between 80:20 and 50:50, more preferably between 75:25 and 65:35, even more preferably 60:40. Even more preferably, the oil consists of at least 90% w/w of Miglyol^{®} 812N. Even more preferably, the oil is Miglyol^{®} 812N.

In another preferred embodiment, optionally in combination with any of the previous or subsequent embodiments, the oil consists of at least 90% w/w of Miglyol^{®} 812N. Even more preferably, the oil is Miglyol^{®} 812N.

In another preferred embodiment, optionally in combination with any of the previous or subsequent embodiments, the oily core consists of at least 90% w/w of Miglyol^{®} 812N.

**In another** preferred embodiment, optionally in combination with any of the previous or subsequent embodiments, the surfactant is an oil-soluble surfactant. Preferably, the oil-soluble surfactant is a phospholipid-based compound selected from the list consisting of lecithin, phosphatidylglycerol, phosphatidylserine, phosphatidylinositol, diphosphatidylglycerol, phosphatidic acid, phosphatidylcholine and phosphatidylethanolamine. Preferably, the phospholipid-based compound is selected from the list consisting of Lipoid S75 (soy phospholipid with 70% phosphatidylcholine), hydrogenated lecithin, phosphatidylcholine, LIPOID S **45** (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%). More preferably, the oil-soluble surfactant is Lipoid S75 (soy phospholipid with 70% phosphatidylcholine).

In another preferred embodiment, optionally in combination with any of the previous or subsequent embodiments, the oil-soluble surfactant is a phospholipid-based compound selected from the list consisting of lecithin, phosphatidylglycerol, phosphatidylserine, phosphatidylinositol, diphosphatidylglycerol, phosphatidic acid, phosphatidylcholine and phosphatidylethanolamine.

In another preferred embodiment, optionally in combination with any of the previous or subsequent embodiments, the phospholipid-based compound is selected from the list consisting of Lipoid S75 (soy phospholipid with 70% phosphatidylcholine), hydrogenated lecithin, phosphatidylcholine, LIPOID S **45** (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%).

In another preferred embodiment, optionally in combination with any of the previous or subsequent embodiments, the phospholipid-based compound is Lipoid S75 (soy phospholipid with 70% phosphatidylcholine).

In another preferred embodiment, optionally in combination with any of the previous or subsequent embodiments, the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, the oil consists of at least 90% w/w of a mixture of caprylic (C8) and capric (C10) triglycerides in a w/w ratio between 80:20 and 50:50, the surfactant is Lipoid S75 (soy phospholipid with 70% phosphatidylcholine), and the nanocapsule system is for the specific use in the prophylactic treatment of an infection caused by the influenza virus.

### DESCRIPTION OF THE FIGURES

**Figure 1****.** Images of the (A) preparation of the aqueous phase and (B) preparation of the nanocapsule emulsion explained in Example 1.
**Figure 2****.** Cell viability curves for the 3 formulations studied.
**Figure 3****.** IL-6 levels in the supernatants of RAW264.7 cells inflamed with LPS prior to administration of the treatment indicated in each case. The levels of significance between the groups are indicated. The correspondence between asterisks and level of significance is: * = α < 0.1, ** = α < 0.01.
**Figure 4****.** Experimental design and treatment of efficacy experiments in mice.
**Figure 5****.** Graph of weight loss over time in mice receiving different types of treatments.
**Figure 6****.** Levels of inflammatory cytokines in the lungs of animals receiving different treatments.
**Figure 7****.** Levels of inflammatory cytokines in the blood of infected animals treated with PBS (control) or our formulation.
**Figure 8****.** Viral load levels in the lungs of animals that received different treatments.
**Figure 9****.** Cellular cytotoxicity assessment by cell metabolic activity, performed in 293-ACE2 cells 3 times in triplicates for 8 dilutions of our nanocapsules (NPs).
**Figure 10****.** RLUs from HIV-1-Luc pseudotyped with SARS-CoV-2 spike in 293-ACE2 cells, where a dose-response effect of the NPs decreasing viral fusion is observed.

### DETAILED DESCRIPTION OF THE EMBODIMENTS

### General considerations

Each embodiment disclosed herein is contemplated as being applicable to each of the other disclosed embodiments. Thus, all combinations of the various elements described herein are within the scope of the invention.

Unless otherwise stated, the terms used in the present application have the meanings indicated herein. Terms that are not defined herein should be given the meanings that would be given to them by a person skilled in the art in the context of this disclosure.

For the purposes of the present invention, any ranges given include both the lower and the upper endpoints of the range.

In the context of the present invention the following general terms are defined:
- The term "comprising" means including, but it is not limited to, whatever follows the word "comprising". Thus, use of the term "comprising" indicates that the listed elements are required or mandatory, but that other elements are optional and may or may not be present. The term "comprising" also includes the term "consists of".
- The term "consisting of" means including, and it is limited to, whatever follows the phrase "consisting of". Thus, the phrase "consisting of" indicates that the listed elements are required or mandatory, and that no other elements may be present.

### Embodiments

In view of the above-mentioned results, a first aspect of the invention refers to a nanocapsule system (hereinafter "nanocapsule system of the invention"), which comprises a surface layer comprising chitosan, for use in the prophylactic treatment of an infection caused by a respiratory pathogen in a subject in need thereof, wherein the nanocapsule is administered to the subject intranasally.

Thus, the first aspect of the invention refers to a nanocapsule system.

The term "nanocapsule system" refers to either an individual nanocapsule or a population of nanocapsules, each having a structure of less than 1um in diameter composed of a core surrounded by a polymeric shell or membrane, forming a well-defined reservoir.

In a preferred embodiment, the nanocapsule system of the invention comprises or consists of: a) a surface layer comprising chitosan, b) an oily core, and c) a surfactant. Preferably, the nanocapsule system is a spherical droplet where the oily core is encapsulated within the chitosan shell, with the surfactant forming a stabilizing boundary at the interface. The nanocapsule system of the invention may be obtained by combining these elements in various ratios, as would be recognized by a person skilled in the art. Specifically, the ratios may range from 0.5% to 20% w/w for the oily core, from 0.05% to 20% w/w for the surfactant, and from 0.01% to 10% w/w for the chitosan surface layer. For instance, the invention can be implemented using the specific quantities of each component exemplified in Table 3.

The term "spherical droplet" refers to a roughly round, three-dimensional structure formed by a liquid or semi-liquid core, maintaining a nearly uniform radius due to surface tension forces, resulting in a smooth, curved outer surface.

The term "encapsulated" refers to the state in which the core material - whether liquid, solid, or gas - is fully enclosed or surrounded by an outer layer or shell, providing protection, controlled release, or functional separation from the surrounding environment.

The term "stabilizing boundary" refers to a functional layer, typically formed by surfactants or emulsifiers, that sits at the interface between two immiscible phases (e.g., oil and water) to reduce surface tension, prevent coalescence, and ensure the structural integrity and longevity of the droplet.

The term "surface layer" refers to the outermost layer of a nanocapsule system, which acts as a protective barrier that surrounds the core material. This layer helps to stabilize the nanocapsule and protect it from environmental factors. In a preferred embodiment, the surface layer comprises or consists of at least 70% w/w of chitosan, preferably at least 80% w/w, more preferably at least 90% w/w. Preferably, the surface layer consists of chitosan.

The term "chitosan" refers to a biocompatible, biodegradable, and non-toxic polysaccharide derived from chitin, composed primarily of β-(1→4)-linked D-glucosamine units, with varying amounts of N-acetyl-D-glucosamine residues. Its properties are typically characterized by parameters such as degree of deacetylation (DDA) and molecular weight (MW). The degree of deacetylation refers to the extent to which the acetyl groups (-COCH3) have been removed from the chitosan molecule. Chitosan is derived from chitin, and the deacetylation process involves removing these acetyl groups, transforming chitin into chitosan. As an expert in the field would recognize, a common method to measure the degree of deacetylation is by using infrared spectroscopy (FTIR). The intensity of the absorption band related to the acetyl group (-C=O stretching at around 1650 cm⁻¹) can be compared to other bands (like the amine group at 1590 cm⁻¹) to determine the amount of deacetylation. Another method involves titration techniques, where the amino group concentration in the chitosan sample is measured and compared to the theoretical maximum amount that would be present if the chitosan were fully deacetylated. On the other hand, the average molecular weight of chitosan refers to the mass of a single molecule of chitosan, averaged over the entire sample. As an expert in the field would recognize, a commonly used technique to determine the molecular weight of chitosan is gel permeation chromatography (GPC), also known as size-exclusion chromatography (SEC). GPC separates molecules based on their size, and by comparing the retention time of the sample with a set of standards of known molecular weight, it is possible to determine the average molecular weight of the chitosan sample. Another method is light scattering or viscometry, where the viscosity of a chitosan solution is measured and related to the molecular weight using specific models.

Importantly, unless otherwise specified in these embodiments, the term 'chitosan' also includes pharmaceutically acceptable salts and functional derivatives of chitosan.

The term "pharmaceutically acceptable salt" refers to a salt form of chitosan that is formed by reacting chitosan with a pharmaceutically acceptable acid, resulting in a compound that is suitable for use in pharmaceutical applications. Such salts must be safe for human or animal use and are typically soluble in water or other suitable solvents, providing enhanced bioavailability or stability without causing adverse effects. These salts are particularly useful in the preparation of chitosan-based nanoparticles or nanocapsules, as they improve the solubility, dispersibility, and stability of chitosan in aqueous environments, which are critical for effective encapsulation, drug delivery, and controlled release. The ability of these salts to solvate chitosan enhances the formation of nanoparticles or nanocapsules, allowing for better encapsulation of active pharmaceutical ingredients (APIs) or bioactive agents. Additionally, the enhanced solubility and stability of these salts in aqueous solutions allow for efficient production of nanocapsules, as well as improved bioavailability and release profiles of the encapsulated substances. Examples of pharmaceutically acceptable salts of chitosan include, but are not limited, to chitosan hydrochloride (chitosan HCl), chitosan lactate, chitosan acetate, chitosan glutamate and chitosan sulfate. These salts facilitate the use of chitosan in various formulations, improving its solubility, stability, and bioavailability in pharmaceutical or therapeutic applications, especially in the preparation of nanocapsules for controlled release and targeted delivery of drugs or bioactive molecules.

The term "functional derivate" refers to a derivative of chitosan in which the chemical structure of the chitosan polymer is modified to introduce specific functional groups or properties. These modifications are intended to enhance the chitosan's characteristics, such as solubility, biocompatibility, charge, or cellular uptake, without altering the fundamental polymer backbone. Such derivatives are particularly useful in the preparation of chitosan-based nanocapsules, as they provide improved properties such as solubility, charge density, and bioactivity, which are essential for the effective encapsulation, stability, and controlled release of therapeutic agents. For example, functional derivatives like quaternized chitosan (N,N,N-trimethyl chitosan chloride - TMC) improve the permeability of nanocapsules and their solubility in aqueous environments, making them ideal for drug delivery applications. Carboxymethyl chitosan (CMC) enhances solubility and introduces a negative charge at physiological pH, which is beneficial for the preparation of stable nanoparticles with targeted delivery capabilities. Hydroxypropyl chitosan improves the bioactivity and water solubility, often used in ophthalmic formulations where high bioavailability and sustained release are critical. Additionally, chitosan oligosaccharides, with their shorter polymer chains, exhibit enhanced bioactivity and are used in therapeutic applications for improved cellular interaction.

These functional derivatives allow chitosan to be tailored for specific applications, particularly in nanoparticle or nanocapsule formulations, providing benefits such as improved solubility, targeted drug delivery, enhanced interaction with biological systems, and optimized release profiles of encapsulated substances. Examples of functional derivatives of chitosan include, but are not limited to quaternized chitosan (N,N,N-trimethyl chitosan chloride - TMC), carboxymethyl chitosan (CMC), hydroxypropyl chitosan and chitosan oligosaccharides.

The examples set in the following section were performed using chitosan HCl (Heppe Medical Chitosan GmbH, Product No. 54040 and 55040). The properties of the specific chitosan batches used in the examples are summarized in **Table 1.**

**Table 1. Properties of the specific chitosan batches used in the examples.**

| **Parameter** | **Limit** | **Result** |
|---|---|---|
| Degree of deacetylation | 75%-90% | 82-83% |
| Molecular weight | To be measured | 109 kDa |
| pH | 4.0-6.0 | 4.1-4.5 |
| Chlorides | 10-20% | 14.9% |
| Protein content | ≤ 0.3% | 0.1-0.2% |

In a preferred embodiment, the chitosan has a degree of deacetylation comprised from 75% to 90% w/w, preferably from 80% to 85% w/w, more preferably 83% w/w.

In a preferred embodiment, the chitosan has an average molecular weight comprised from 90 kDa to 120 kDa, preferably from 100 kDa to 120 kDa, more preferably from 104 kDa to 114 kDa, even more preferably 109 kDa.

In another preferred embodiment, the chitosan has a pH comprised from 4.0 to 6.0, preferably from 4.0 to 5.0, more preferably from 4.1 to 4.5.

In a preferred embodiment, the chitosan is in its native chemical structure. The term "native chemical structure" refers to the unaltered polymeric backbone and natural functional groups of chitosan, free from covalent chemical modifications such as sulfation, sulfonation, or other substitutions that change the primary molecular structure. This term includes chitosan in its free base form, protonated form, or pharmaceutically acceptable salts resulting from ionic interactions - such as chitosan hydrochloride - provided these forms retain the original backbone and functional integrity of the chitosan molecule. However, this term does not encompass functional derivatives of chitosan, such as quaternized chitosan, carboxymethyl chitosan, or other modified forms that introduce additional functional groups or alter the chitosan's chemical structure.

Preferably, the chitosan is a chitosan salt. More preferably, the chitosan is a chitosan salt selected from the list consisting of chitosan hydrochloride (chitosan HCl), chitosan lactate, chitosan acetate, chitosan glutamate and chitosan sulfate. Even more preferably the chitosan is chitosan hydrochloride. Even more preferably, the chitosan hydrochloride a percentage of chloride comprised from 10% to 20% w/w, preferably from 13% to 17% w/w, more preferably from 14% to 16% w/w, even more preferably 14.9%.

In another preferred embodiment, the chitosan has a protein content is equal or lower than 0.3% w/w, preferably 0.2% w/w.

Other embodiments related to the properties of chitosan set below. Please note that all the embodiments of the first aspect of the invention apply herein:
- In a particular preferred embodiment the chitosan has a degree of deacetylation comprised from 75% to 90% w/w, preferably from 80% to 85% w/w, more preferably 83% w/w, and an average molecular weight comprised from 90 kDa to 120 kDa, preferably from 100 kDa to 120 kDa, more preferably from 104 kDa to 114 kDa, even more preferably 109 kDa.
- In a particular preferred embodiment the chitosan has a degree of deacetylation comprised from 80% to 85% w/w, more preferably 83% w/w, and an average molecular weight comprised from 100 kDa to 120 kDa, more preferably from 104 kDa to 114 kDa, even more preferably 109 kDa.
- In a particular preferred embodiment the chitosan has a degree of deacetylation comprised from 80% to 85% w/w, more preferably 83% w/w, and an average molecular weight comprised from 104 kDa to 114 kDa, even more preferably 109 kDa.
- In a particular preferred embodiment the chitosan has a degree of deacetylation comprised from 75% to 90% w/w, preferably from 80% to 85% w/w, more preferably 83% w/w, an average molecular weight comprised from 90 kDa to 120 kDa, preferably from 100 kDa to 120 kDa, more preferably from 104 kDa to 114 kDa, even more preferably 109 kDa, and is in its native chemical structure.
- In a particular preferred embodiment the chitosan has a degree of deacetylation comprised from 80% to 85% w/w, more preferably 83% w/w, an average molecular weight comprised from 100 kDa to 120 kDa, more preferably from 104 kDa to 114 kDa, even more preferably 109 kDa, and is in its native chemical structure.
- In a particular preferred embodiment the chitosan has a degree of deacetylation comprised from 80% to 85% w/w, more preferably 83% w/w, an average molecular weight comprised from 104 kDa to 114 kDa, even more preferably 109 kDa, and is in its native chemical structure.

The term "oily core" refers to the hydrophobic, oil-based interior of a nanocapsule or similar encapsulated structure, designed to solubilize, protect, and deliver lipophilic active ingredients. This core is typically composed of one or more oils, which can include natural, synthetic, or semi-synthetic lipids, such as triglycerides, fatty acid esters, essential oils, hydrocarbons, or silicone oils. The oily core serves as a reservoir that helps encapsulate poorly water-soluble compounds, enhancing their stability, bioavailability, or controlled release. The specific composition of the oily core can be tailored to the desired physical and chemical properties of the formulation, including viscosity, polarity, and compatibility with other components like surfactants or polymers that form the outer shell of the nanocapsule.

The examples set in the following section were performed using an oily core of Miglyol^{®} 812N. Miglyol^{®} 812N is a medium-chain triglyceride oil composed of a mixture of caprylic acid (C8:0) 50-80% and capric acid (C10:0) 20-50%, typically derived from coconut or palm kernel oil. The standarized ratio for Miglyol^{®} 812N is a 60:40 w/w ratio of caprylic acid (C8:0) to capric acid (C10:0).

In a preferred embodiment, the oily core comprises or consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, any synthetic or semi-synthetic derivatives and combinations thereof. Preferably, the oily core comprises or consists of triglycerides with three fatty acids having an aliphatic tail of 6 to 12 carbon atoms. More preferably, the oily core comprises or consists of a mixture of caprylic (C8) and capric (C10) triglycerides. Preferably, the mixture of caprylic (C8) and capric (C10) triglycerides is in a w/w ratio between of 80:20 and 50:50, more preferably between 75:25 and 65:35, even more preferably 60:40. Even more preferably, the oily core comprises or consists of at least 90% w/w of Miglyol^{®} 812N. Even more preferably, the oil consists of Miglyol^{®} 812N.

The term "oil" refers to refers to any organic, lipid-based substance that is liquid or semi-solid at room temperature, primarily composed of hydrocarbons, fatty acids, esters, fatty alcohols, or their derivatives. It can originate from plant, animal, mineral, synthetic, or semi-synthetic sources, and is typically hydrophobic and immiscible with water, serving functions such as lubrication, emolliency, nutrient delivery, fragrance, or chemical solvency.

In a preferred embodiment, the oil comprises or consists of at least 50%, 60%, 70%, 80% or 90% w/w of triglycerides with three fatty acids having an aliphatic tail of 6 to 12 carbon atoms. Preferably, the oil comprises or consists of at least 90% w/w of triglycerides with three fatty acids having an aliphatic tail of 6 to 12 carbon atoms. Even more preferably, the oil comprises or consists of at least 90% w/w of a mixture of caprylic (C8) and capric (C10) triglycerides. Preferably, the mixture of caprylic (C8) and capric (C10) triglycerides is in a w/w ratio between 80:20 and 50:50, more preferably between 75:25 and 65:35, even more preferably 60:40. Even more preferably, the oil comprises or consists of at least 90% w/w of Miglyol^{®} 812N.

The term "long-chain" mono-, di-, or triglycerides refers to lipids composed of a glycerol molecule attached to one, two, or three long-chain fatty acids, typically containing 13 or more carbon atoms (commonly between C14 and C22). These lipids are prevalent in natural fats and oils and are widely used in the pharmaceutical, cosmetic, and food industries for their emulsifying and biocompatible properties. Examples of long-chain monoglycerides include glyceryl monolinoleate (C18:2), glyceryl monooleate (C18:1), and glyceryl monostearate (C18:0), with commercial products such as Maisine^{®} CC (glyceryl monolinoleate) and Peceol^{™} (glyceryl monooleate). Long-chain diglycerides include glyceryl dioleate, glyceryl distearate, and glyceryl dilinoleate, with commercial examples like Capmul^{®} (various diglycerides used as emulsifiers and solubilizers). Long-chain triglycerides include triolein (glyceryl trioleate, C18:1), trilinolein (glyceryl trilinoleate, C18:2), and tristearin (glyceryl tristearate, C18:0), and naturally occurring oils like soybean oil, corn oil, and safflower oil, commonly employed in emulsions and lipid nanoparticle formulations.

The term "medium-chain" mono-, di-, or triglycerides refers to lipids composed of a glycerol molecule attached to one, two, or three medium-chain fatty acids, typically containing 6 to 12 carbon atoms, such as caproic acid (C6), caprylic acid (C8), capric acid (C10), and lauric acid (C12). These lipids are known for their rapid digestion and absorption, making them useful in nutritional, pharmaceutical, and cosmetic applications. Examples of medium-chain monoglycerides include glyceryl monocaprylate (C8), glyceryl monocaprate (C10), and glyceryl monolaurate (C12), with commercial products such as Capmul^{®} MCM (glyceryl monocaprylate) and Lauroglycol^{™} (glyceryl monolaurate), commonly used as solubilizers and emulsifiers. Examples of medium-chain diglycerides include glyceryl dicaprylate, glyceryl dicaprate, and glyceryl dilaurate, with commercial products like Captex^{®} 200 (a blend of medium-chain diglycerides) and Capmul^{®} 808DG (glyceryl dicaprylate/dicaprate), which are widely employed in lipid-based formulations for enhancing drug delivery and bioavailability. Natural sources rich in medium-chain triglycerides include coconut oil, palm kernel oil, and dairy fats such as butter and goat's milk. Some commercial examples include Miglyol^{®} products (including Miglyol^{®} 812N, Miglyol^{®} 810 N, and Miglyol^{®} 840 N) and Labrafac^{®} products (such as Labrafac^{®} Lipophile WL 1349 and Labrafac^{®} CC), which are essentially consist of a mixture of triglycerides of caprylic (C8) and capric (C10) acids.

The term "triglycerides with three fatty acids having an aliphatic tail of 6 to 12 carbon atoms", generally referred to as medium-chain triglycerides (MCTs), refers to a type of glyceride molecule composed of a glycerol backbone linked through ester bonds to three fatty acids, each containing a straight-chain hydrocarbon tail with 6 to 12 carbon atoms. These fatty acids include caproic acid (C6:0), caprylic acid (C8:0), capric acid (C10:0), and lauric acid (C12:0). Examples of triglycerides formed from these fatty acids include tricaproin (from caproic acid), tricaprylin (from caprylic acid), tricaprin (from capric acid), and trilaurin (from lauric acid). Natural sources rich in these triglycerides include coconut oil, palm kernel oil, and dairy fats such as butter and goat's milk. Some commercial examples include Miglyol^{®} products (including Miglyol^{®} 812N, Miglyol^{®} 810 N, and Miglyol^{®} 840 N) and Labrafac^{®} products (such as Labrafac Lipophile WL 1349 and Labrafac CC), which are essentially consist of a mixture of triglycerides of caprylic (C8) and capric (C10) acids. As an expert in the field would recognize, several analytical techniques can be used to determine if an oil meets the characteristic of comprising triglycerides with three fatty acids having an aliphatic tail of 6 to 12 carbon atoms. First, gas chromatography (GC) is commonly employed to analyze the fatty acid composition of the oil. The oil sample is converted into fatty acid methyl esters (FAMEs), which are then separated based on their molecular size and structure. By comparing the retention times of the peaks to standards, it is possible to identify fatty acids with chain lengths ranging from 6 to 12 carbon atoms, such as caproic acid (C6), caprylic acid (C8), and lauric acid (C12). In combination with mass spectrometry (MS), which can be coupled with GC (GC-MS), the molecular weight and structure of the fatty acids can be further confirmed. High-performance liquid chromatography (HPLC) can also be used for a similar purpose, providing an alternative method for separating and identifying the fatty acids, although it is generally more suitable for less volatile compounds than those analyzed by GC. Additionally, nuclear magnetic resonance (NMR) spectroscopy can be applied to determine the exact structure of the fatty acids in the triglycerides by analyzing the chemical shifts and splitting patterns in the NMR spectra, providing detailed information about the carbon chain length and the presence of any unsaturation or branching in the fatty acids. Finally, the oil's composition can be further confirmed through fatty acid profiling, which involves a combination of these techniques to give a complete overview of the fatty acid content and verify the presence of triglycerides with the desired aliphatic chains. These techniques would also allow us to determine the specific percentage of each type of triglyceride in an oil.

The term "surfactant" refers to an amphiphilic molecule that reduces surface tension between two immiscible phases, typically a liquid and a solid or between two liquids. Surfactants possess both hydrophilic and hydrophobic components, allowing them to adsorb at interfaces and stabilize dispersions. Surfactants can be broadly categorized based on their solubility and ionic nature. In terms of solubility, surfactants are classified as either water-soluble or oil-soluble. Regarding ionic nature, they are grouped into anionic (negatively charged), cationic (positively charged), nonionic (no charge), and amphoteric or zwitterionic (both positive and negative charges) surfactants. In the context of the present invention, the term "surfactant" is also intended to cover emulsifying components with surfactant-like properties, such as phospholipids with surfactant-like properties. Examples of such emulsifying components that may not be traditionally considered as surfactants include phospholipids like Lipoid S75, which is derived from soybean lecithin and contains approximately 70% phosphatidylcholine. Other examples are natural emulsifiers such as egg yolk (rich in lecithin), certain proteins with emulsifying properties like casein or whey proteins, modified starches with amphiphilic characteristics, saponins (plant-derived compounds with surfactant-like properties), and certain polymers with both hydrophilic and hydrophobic segments, such as some cellulose derivatives. These components, while not classical surfactants, can function similarly in stabilizing emulsions, reducing interfacial tension, and facilitating the mixing of otherwise immiscible substances.

The examples set in the following section were performed using Lipoid S75 as a surfactant. Lipoid S75 is a soy lecithin-based phospholipid product, primarily composed of around 70% phosphatidylcholine. Lipoid S75 is a phospholipid-based compound that functions as an oil-soluble surfactant.

In a preferred embodiment, the surfactant is an oil-soluble surfactant.

The term "oil-soluble surfactant" refers to a surfactant that has a stronger affinity for oil than water due to its molecular structure, which typically features a larger hydrophobic portion compared to their hydrophilic component. They typically have a low Hydrophilic-Lipophilic Balance (HLB) value, generally between 3 and 6, which reflects their lipophilic nature. They are designed to stabilize water-in-oil emulsions and are widely used in formulations where the continuous phase is oil-based. Their hydrophobic portion interacts with the oil phase, while their limited hydrophilic components allow them to function effectively in non-aqueous systems. Examples of oil-soluble surfactants, including some oil-soluble emulsifying components with surfactant-like properties include: sorbitan esters - such as sorbitan monooleate (Span 80), sorbitan monolaurate (Span 20), sorbitan monostearate (Span 60), and sorbitan tristearate (Span 65) -, polyglycerol esters - such as polyglycerol polyricinoleate (PGPR) and polyglycerol oleate -, glyceryl esters (such as glyceryl monooleate and glyceryl monostearate), propylene glycol esters - such as propylene glycol monocaprylate (Capryol PGMC) and propylene glycol monolaurate -, polyglyceryl fatty acid esters - such as polyglyceryl-3 diisostearate and polyglyceryl-4 oleate -, sucrose esters (such as sucrose distearate and sucrose tristearate), fatty acid esters (such as isopropyl myristate, isopropyl palmitate, cetyl palmitate, and butyl stearate), dioctyl sodium sulfosuccinate (AOT), polyoxyethylene sorbitol hexaoleate, lanolin alcohols and derivatives (such as lanolin alcohol, acetylated lanolin alcohol, and hydrogenated lanolin), certain fatty acid salts with long hydrocarbon chains (between 12 and 22 carbon atoms) - such as calcium and magnesium salts of lauric acid (C12), myristic acid (C14), palmitic acid (C16), stearic acid (C18), and behenic acid (C22) -, and phospholipid-based compounds, such as Lipoid S75 (soy phospholipid with 70% phosphatidylcholine).

Preferably, the oil-soluble surfactant is selected from the list consisting of sorbitan esters, polyglycerol esters, glyceryl esters, propylene glycol esters, polyglyceryl fatty acid esters, sucrose esters, fatty acid esters, dioctyl sodium sulfosuccinate (AOT), polyoxyethylene sorbitol hexaoleate, lanolin alcohols and derivatives, fatty acid salts with hydrocarbon chains of 12 to 22 carbon atoms, and phospholipid-based compounds.

More preferably, the oil-soluble surfactant is a phospholipid-based compound.

Even more preferably, the oil-soluble surfactant is Lipoid S75 (soy phospholipid with 70% phosphatidylcholine).

The term "phospholipid-based compound" refers to a class of molecules derived from or structurally similar to phospholipids, which are amphiphilic lipids consisting of a hydrophilic head containing a phosphate group and two hydrophobic tails derived from fatty acids, typically joined by a glycerol backbone. These compounds retain the fundamental **characteristics** of phospholipids, including their ability to form bilayers and their importance in biological membranes. Phospholipid-based compounds may include natural phospholipids like phosphatidylcholine (lecithin), phosphatidylethanolamine, and phosphatidylserine, as well as modified or synthetic variants designed for specific applications in areas such as drug delivery, nanotechnology, and materials science.

In a preferred embodiment, the phospholipid-based compound is selected from the list consisting of lecithin, phosphatidylglycerol, phosphatidylserine, phosphatidylinositol, diphosphatidylglycerol, phosphatidic acid, phosphatidylcholine and phosphatidylethanolamine.

Preferably, the phospholipid-based compound is selected from the list consisting of Lipoid S75 (soy phospholipid with 70% phosphatidylcholine), hydrogenated lecithin, phosphatidylcholine, LIPOID S 45 (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%).

More preferably, the phospholipid-based compound is Lipoid S75 (soy phospholipid with 70% phosphatidylcholine).

The nanocapsule system of the invention has an intrinsic prophylactic effect even in the absence of active ingredients. The examples set in the following section were performed using nanocapsules that are neither functionalized nor loaded with active ingredients. Instead, they possess an inherent prophylactic effect resulting solely from the combination of the nanocapsules' components.

In a preferred embodiment, the nanocapsule system comprises an empty oily core, devoid of active ingredients. Preferably, the nanocapsule system further comprises an empty surface layer, devoid of active ingredients.

The term "empty oily core" refers to the internal compartment of the nanocapsule that consists of oils or lipids but does not contain any active ingredient or bioactive compound. This means that the oily core functions primarily to stabilize the structure of the nanocapsule, providing a medium for encapsulation without any therapeutic or active compounds being loaded. To verify whether a nanocapsule has an empty oily core, methods such as Gas Chromatography (GC), Mass Spectrometry (MS), and Differential Scanning Calorimetry (DSC) are commonly used. GC can separate and identify individual components in the oily core, while MS provides detailed molecular information, confirming the absence of any active compounds. DSC measures the thermal properties of the nanocapsule and can help assess the nature of the oily core, ensuring no active ingredient is present. Additionally, an expert in the field will typically know whether a nanocapsule contains an active ingredient by consulting the product's datasheet, which specifies the components and composition used in the formulation. This allows the expert to verify the presence or absence of active ingredients based on the raw materials used.

The term "active ingredient" refers to a substance that provides a therapeutic, chemical, physical, or biological effect in the nanocapsule system. This could include drugs, vitamins, antioxidants, or other compounds with a functional impact. In the context of the present invention, the term specifically excludes any components that are part of the nanocapsule system of the invention, regardless of whether they present inherent antimicrobial or protective properties, as well as any elements that may result from the combination of those components. Therefore, neither the chitosan surface layer, nor the oils that comprise the oily core, nor the surfactant are considered "active ingredients" in this context. To determine whether a nanocapsule contains an active ingredient, methods like High-Performance Liquid Chromatography (HPLC), UV-Vis Spectroscopy, and Liquid Chromatography-Mass Spectrometry (LC-MS) are typically employed. HPLC separates and quantifies the active compounds, while UV-Vis spectroscopy can detect specific active ingredients based on their characteristic absorbance. LC-MS, which combines the separation capabilities of liquid chromatography with the identification power of mass spectrometry, is especially useful for identifying and quantifying active ingredients. Experts in the field will typically know whether a nanocapsule has an active ingredient by referring to the datasheet of the materials used in the formulation. The datasheet will list any active compounds incorporated into the nanocapsules, allowing experts to confirm their presence or absence in the final product.

The term "empty surface layer" refers to the external coating of the nanocapsule that does not contain any active ingredients. This means the surface is free from any agents intended to provide therapeutic effects, regardless of the presence or absence of functional groups. To determine whether a nanocapsule has an empty surface layer, X-ray Photoelectron Spectroscopy (XPS) and Fourier Transform Infrared Spectroscopy (FTIR) can be used. XPS can analyze the elemental composition and detect the presence of any surface elements, while FTIR can provide insights into the molecular vibrations of the surface layer, confirming the absence of active ingredients. Experts in the field can also consult the datasheet of the nanocapsule components to verify whether any active ingredients were incorporated into the surface layer during production.

In a preferred embodiment, the nanocapsule system exhibits an intrinsic prophylactic effect in the absence of active ingredients.

The term "intrinsic" refers to a property or effect that is naturally inherent to the nanocapsule system itself - arising from the specific combination of its components - rather than being caused by an added active ingredient. It implies that the nanocapsule's prophylactic (protective) effect comes from the materials and structure of the nanocapsule alone, without relying on external therapeutic compounds. To assess whether a nanocapsule presents an intrinsic prophylactic effect in the absence of active ingredients, several approaches could be employed, including:
- *In vitro* studies: Cell culture models using respiratory epithelial cells can be exposed to both the nanocapsule system and a respiratory pathogen. A reduction in pathogen attachment or cell infection - compared to a control group without nanocapsules - would suggest an intrinsic protective effect.
- Animal models: *In vivo* experiments can assess whether subjects treated with the nanocapsules exhibit lower infection rates or less severe symptoms when exposed to respiratory pathogens - even without an active ingredient.
- Biochemical analysis: Measuring inflammatory markers, cytokine production, or other immune responses after exposure to the nanocapsules can help determine whether they elicit a protective effect independent of active compounds.

An expert in the field would typically design these tests based on the nanocapsule's composition and refer to the datasheets of the materials involved to ensure no hidden active ingredients are contributing to the effect.

Regarding the physical and chemical properties of the nanocapsule system, the size of the nanocapsule plays an important role in determining its biological interactions, cellular uptake, and circulation time in the body, with smaller sizes generally promoting better cellular internalization and longer systemic circulation. Additionally, factors such as pH, the polydispersity index (Pdl), and zeta potential (Zpot) influence the stability, permeability, and release behavior of nanocapsules. pH affects the ionization state of the shell and core, altering interactions with the environment. Pdl refers to the distribution of particle sizes in a sample, with a lower Pdl indicating a more uniform size distribution and a higher Pdl reflecting a broader range of particle sizes. Zpot determines colloidal stability, with higher absolute values indicating better suspension stability and reduced aggregation.

In a preferred embodiment, the nanocapsules of the nanocapsule system of the invention have a mean diameter of between 1 and 999 nm, preferably between 50 and 500 nm, more preferably between 100 and 400 nm, even more preferably between 200 and 300 nm, even more preferably between 230 and 270 nm.

The term "mean diameter" refers to the diameter measured by means of the Dynamic Light Scattering (DLS) technique, which is defined by the hydrodynamic diameter of a sphere which scatters at the same speed as the particles being measured. The size of the nanocapsules is primarily influenced by the composition and formation conditions and can be measured using standard methods known by the person skilled in the art and described in the examples section.

In another preferred embodiment, the nanocapsules of the nanocapsule system of the invention have a pH of between 3.9 and 5.1, preferably between 4.1 and 4.9, more preferably between 4.3 and 4.7.

In another preferred embodiment, the nanocapsules of the nanocapsule system of the invention have a polydispersity index (Pdl) of between 0.05 and 0.35, preferably between 0.10 and 0.30, more preferably between 0.15 and 0.25.

In another preferred embodiment, the nanocapsules of the nanocapsule system of the invention have a zeta potential (Zpot) of between 5 mV and 45 mV, preferably between 15 mV and 35 mV.

In another preferred embodiment, the nanocapsules of the nanocapsule system of the invention have a pH of between 4.3 and 4.7, a polydispersity index (Pdl) of between 0.15 and 0.25, and a zeta potential (Zpot) of between 15 mV and 35 mV.

The nanocapsule system of the invention is for the specific use in the prophylactic treatment of an infection.

The term "prophylactic treatment" refers to a medical or therapeutic intervention intended to prevent the occurrence or onset of a disease or condition, rather than treating or curing it after it has developed. Prophylactic treatment is preferably administered before any clinical symptoms appear, aiming to reduce the risk of developing the disease in the first place. In contrast, therapeutic treatment is designed to manage or cure a disease after clinical symptoms have already emerged. While prophylactic approaches focus on prevention, therapeutic treatments address an existing condition, aiming to alleviate symptoms, control progression, or achieve a cure. The key difference lies in timing and purpose: prophylactic treatment is preventive and proactive, preferably applied before the onset of clinical signs, whereas therapeutic treatment is reactive, initiated after symptoms are present.

The nanocapsule system of the invention is for the specific use in the prophylactic treatment of an infection caused by a respiratory pathogen.

The term 'infection caused by a respiratory pathogen' refers to a disease or infection that occurs as a result of the invasion and multiplication of a respiratory pathogen (see definition below) that primarily targets and affects the respiratory system, which includes the nasal passages, throat, lungs, and airways. Examples of infections caused by respiratory pathogens include influenza (caused by the influenza virus), COVID-19 (caused by the coronavirus SARS-CoV-2), pneumonia (which can be caused by *Streptococcus pneumoniae, Haemophilus influenzae,* or viruses like respiratory syncytial virus), tuberculosis (caused by *Mycobacterium tuberculosis),* and fungal infections such as aspergillosis (caused by *Aspergillus species).*

The term "respiratory pathogen" refers to any microorganism that is capable of causing disease or infection in the respiratory system, including viruses (such as influenza, coronavirus, and respiratory syncytial virus), bacteria (such as *Streptococcus pneumoniae* and *Mycobacterium tuberculosis),* and fungi (such as *Aspergillus* species).

In a preferred embodiment, the respiratory pathogen is selected from the group consisting of a virus, a bacterium, or a fungus. Preferably, the respiratory pathogen is a respiratory virus. Preferably, the respiratory virus is selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses (e.g. HCoV-229E, HCoV-NL63, HCoV-OC43 and HCoV-HKU1), bocavirus, and enteroviruses (e.g. coxsackieviruses and echoviruses).

In a preferred embodiment, the respiratory virus is the influenza virus, preferably an influenza A virus, more preferably H1N1 influenza.

The term "influenza virus" refers to a group of viruses belonging to the Orthomyxoviridae family, which are responsible for causing the infectious disease known as influenza, affecting the respiratory system. Influenza viruses are classified into several types, including A, B, and C, with Type A being the most common cause of seasonal flu epidemics.

The term "influenza A virus" refers to a subtype of the influenza virus that is responsible for most seasonal flu epidemics and pandemics. Influenza A viruses can infect humans as well as a variety of animals, including birds and pigs. This type of virus is further classified based on its surface proteins, hemagglutinin (H) and neuraminidase (N). Examples of influenza A virus subtypes include H1N1, which caused the 2009 swine flu pandemic, and H3N2, which is commonly associated with seasonal flu outbreaks in humans.

The term "H1N1 influenza" refers to a specific strain of the influenza A virus characterized by the H1 and N1 subtypes of hemagglutinin and neuraminidase, respectively. This strain gained widespread attention during the 2009 swine flu pandemic and is a common cause of seasonal influenza. The H1N1 strain is closely related to the PR8 strain (A/Puerto Rico/8/34), which is a laboratory-adapted virus used in vaccine development and research. The PR8 strain is an early isolate of the H1N1 virus and shares similar genetic and antigenic properties, serving as a model for studying influenza A virus infections and vaccine responses.

In another preferred embodiment, the respiratory virus is a coronavirus, preferably of the *Betacoronavirus* genus, more preferably of the *Severe acute respiratory syndrome-related coronavirus* species, more preferably selected from SARS-CoV-2 an SARS-CoV, even more preferably SARS-CoV-2.

The term "coronavirus" refers to any virus belonging to the family *Coronaviridae,* which consists of enveloped, positive-sense, single-stranded RNA viruses characterized by their crown-like (corona) appearance under electron microscopy due to spike (S) glycoproteins on their surface. Coronaviruses are classified into four genera: *Alphacoronavirus, Betacoronavirus, Gammacoronavirus,* and *Deltacoronavirus.* While *Alphacoronavirus* and *Betacoronavirus* primarily infect mammals, including humans, *Gammacoronavirus* and *Deltacorona virus* mainly infect birds.

The term *"Betacoronavirus* genus" refers to a virus belonging to the genus *Betacoronavirus,* which is part of the *Coronaviridae* family. *Betacoronavirus* includes several species of enveloped, positive-sense, single-stranded RNA viruses that primarily infect mammals and can cause respiratory, enteric, hepatic, and neurological diseases. Notable human-infecting *Betacoronaviruses* include SARS-CoV, SARS-CoV-2, MERS-CoV, HCoV-OC43, and HCoV-HKU1.

The term *"Severe acute respiratory syndrome-related coronavirus"* refers to a viral species within the genus *Betacoronavirus* and the family *Coronaviridae.* This species includes multiple coronaviruses capable of infecting humans and animals, including SARS-CoV and SARS-CoV-2, which are associated with severe respiratory diseases. These viruses share genetic and structural similarities and utilize the angiotensin-converting enzyme 2 (ACE2) receptor for host cell entry.

The term "SARS-CoV virus" refers to the specific strain of coronavirus identified as Severe Acute Respiratory Syndrome Coronavirus (SARS-CoV), which caused the 2002-2003 SARS outbreak. SARS-CoV belongs to the species *Severe acute respiratory syndrome-related corona virus within* the genus *Betacoronavirus* and the family *Coronaviridae.* It is an enveloped, positive-sense, single-stranded RNA virus that primarily causes severe respiratory illness in humans.

The term "SARS-CoV-2 virus" refers to the specific strain of coronavirus identified in 2019 as Severe Acute Respiratory Syndrome Coronavirus 2 (SARS-CoV-2), responsible for the COVID-19 pandemic. SARS-CoV-2 belongs to the species *Severe acute respiratory syndrome-related coronavirus* within the genus *Betacoronavirus* and the family *Coronaviridae.* It shares genetic and structural similarities with SARS-CoV but has distinct epidemiological and pathogenic characteristics. SARS-CoV-2 primarily spreads through respiratory droplets and utilizes the ACE2 receptor for host cell entry.

The nanocapsule system of the invention is for the specific use in the prophylactic treatment of a subject in need.

The term "subject in need" refers to an individual who is at risk of contracting or developing a disease (in this case an infection caused by a respiratory pathogen) and who may benefit from prophylactic treatment to prevent such an infection. This includes individuals who are more vulnerable to respiratory infections due to factors such as age, pre-existing health conditions, immunocompromised states, or exposure to environments with a higher risk of pathogen transmission. Examples of subjects in need include the elderly, young children, individuals with chronic respiratory conditions (such as asthma or chronic obstructive pulmonary disease), individuals with weakened immune systems (e.g., those undergoing chemotherapy or living with HIV), healthcare workers exposed to respiratory pathogens, and individuals in high-risk environments such as nursing homes or hospitals.

In a preferred embodiment, the subject in need is a mammal, preferably a human.

The nanocapsule system of the invention is administered to the subject intranasally.

The term "intranasally" refers to a method of administering a substance through the nose, typically via sprays, drops, or aerosols. The substance is absorbed through the nasal mucosa, providing a route of entry into the body.

As mentioned above, the nanocapsule system of examples set in the following section do not act as adjuvants, modulators, or delivery vehicles for other active compounds. Instead, they possess an inherent prophylactic effect.

In a preferred embodiment, the prophylactic treatment comprises or consists of preventing the respiratory pathogen from causing a symptomatic infection, preferably by preventing the respiratory pathogen from entering the nasal epithelium by forming a protective barrier.

The term "preventing the respiratory pathogen from causing a symptomatic infection" refers to a process or intervention aimed at stopping a respiratory pathogen (as defined above) from progressing to a state where it produces clinical symptoms. This could involve inhibiting the pathogen's replication, reducing its spread within the respiratory tract, or mitigating its ability to trigger an immune response that leads to symptoms such as coughing, sneezing, or inflammation. As an expert in the field would recognize, several methods can be employed individually or in combination to evaluate whether a prophylactic treatment effectively prevents a respiratory pathogen from causing a symptomatic infection:
- *In vitro* cell culture assays: These involve exposing respiratory epithelial cells to the pathogen in the presence or absence of the prophylactic treatment. Researchers can measure the pathogen's replication rate and assess cell viability using techniques such as plaque assays, quantitative PCR (qPCR), or immunofluorescence microscopy to determine whether the treatment limits viral or bacterial proliferation, reducing the likelihood of symptom development.
- Cytokine and inflammatory marker analysis: Since symptomatic infections often arise from the host's immune response to the pathogen, assays such as enzyme-linked immunosorbent assays (ELISA) or multiplex cytokine profiling can measure the levels of pro-inflammatory cytokines (e.g., IL-6, TNF-α). A reduction in these markers suggests that the treatment helps prevent the immune cascade leading to symptoms.
- Animal models: *In vivo* studies using animals can assess whether the prophylactic treatment reduces both pathogen load and the onset of observable symptoms, such as respiratory distress or weight loss. Comparison of treated and untreated groups helps determine if the treatment prevents symptomatic infection.
- Clinical symptom scoring: In human or animal studies, researchers can monitor for predefined clinical signs (e.g., nasal discharge, fever, or respiratory difficulty) alongside pathogen load assessments. A reduction or absence of such symptoms in the treatment group would indicate that the prophylactic approach is effective at preventing symptomatic infection.
- Molecular assays: Polymerase chain reaction (PCR) or RNA sequencing can be used to quantify the pathogen's genetic material in respiratory tissues. Coupled with symptom monitoring, a reduction in both pathogen load and associated inflammatory gene expression supports the conclusion that the treatment prevents symptomatic infection.

The term "preventing the respiratory pathogen from entering the nasal epithelium" refers to a process or intervention aimed at stopping a respiratory pathogen (as defined above) from attaching to or penetrating the cells lining the nasal passages (the nasal epithelium). This could involve blocking the pathogen's ability to bind to receptors or preventing its transport into the cells, thereby preventing infection. As an expert in the field would recognize, several methods can be employed individually or in combination to evaluate whether a prophylactic treatment effectively prevents a respiratory pathogen from entering the nasal epithelium:
- *In vitro* cell culture assays: These involve exposing cultured nasal epithelial cells to the pathogen in the presence or absence of the prophylactic treatment. The ability of the pathogen to bind to the cells or invade them can be measured using techniques such as flow cytometry or immunofluorescence microscopy.
- Binding inhibition assays: These assays assess whether the prophylactic treatment can block the pathogen from binding to specific receptors on the nasal epithelium. Techniques like enzyme-linked immunosorbent assays (ELISA) or surface plasmon resonance can help quantify the extent of binding inhibition.
- Animal models: *In vivo* studies using animals can help evaluate the effect of the treatment on pathogen colonization in the nasal cavity. By comparing treated and untreated animals, researchers can determine if the treatment reduces pathogen presence in the nasal epithelium.
- Molecular assays: Polymerase chain reaction (PCR) or RNA sequencing can be used to detect the presence of the pathogen in the nasal epithelium after treatment. A reduction in the pathogen's genetic material would indicate that the treatment has successfully blocked its entry.

The term "protective barrier" refers to a mechanism that prevents or reduces the entry of pathogens into the nasal epithelial cells. Possible protective barrier mechanisms may include: a physical barrier (a layer that physically blocks pathogens from reaching the nasal epithelium, such as a coating or film that prevents direct contact), a chemical barrier (substances that may alter the environment in a way that inhibits pathogens, such as altering pH or interfering with pathogen attachment), natural defense modulation (enhancement of the body's immune defenses, potentially by boosting mucus production or stimulating immune responses at the nasal surface), an enzymatic barrier (enzymes or substances that modulate enzymatic activity, enhancing the degradation or neutralization of pathogens before they can penetrate the nasal epithelium), or a hydration barrier (a moisture layer that could trap pathogens or facilitate their removal from the nasal passages).

In a preferred embodiment, the protective barrier is a physical barrier, such as a polymeric coating that prevents direct contact of pathogens with the nasal epithelium.

The term "physical barrier" refers to a layer that forms a continuous, uninterrupted coating over the nasal epithelium, preventing direct contact between pathogens and epithelial cells. As a person skilled in the art would recognize, this can be evaluated by visualizing the nanocapsule layer using electron microscopy to confirm the formation of a continuous coating over the epithelium and by testing pathogen adhesion reduction *in vitro.* An indicative threshold of effectiveness for a physical barrier would be a coating coverage of at least 95% of the epithelial surface, with pathogen adhesion reduced by at least 80% compared to untreated controls.

In another preferred embodiment, the protective barrier is a chemical barrier, such as a substance that alters the nasal pH to inhibit pathogen attachment or neutralize pathogens.

The term "chemical barrier" refers to a substance that alters the nasal pH or other chemical properties in a way that inhibits pathogen attachment or neutralizes pathogens. As a person skilled in the art would recognize, this can be determined by measuring pH changes after nanocapsule application and assessing pathogen viability or attachment in environments mimicking nasal conditions. An indicative threshold for a chemical barrier would be a pH shift of at least 0.5 units from the baseline nasal pH (typically around 6.0 to 6.5) and a reduction in pathogen viability or attachment by at least 75% in vitro.

In another preferred embodiment, the protective barrier enhances natural defense mechanisms, such as by stimulating increased mucus production or activating local immune cells.

The term "enhances natural defense mechanisms" refers to the stimulation of innate immune responses, including increased mucus production or activation of local immune cells, which helps trap and expel pathogens. As a person skilled in the art would recognize, this can be verified by analyzing mucus secretion levels and cytokine production in cell cultures or animal models treated with the nanocapsule system. An indicative threshold for enhancing natural defenses would be a 50% or greater increase in mucus production (e.g., mucin levels) and a statistically significant rise (p<0.05) in pro-inflammatory cytokines like IL-6 or IL-8 compared to baseline.

In another preferred embodiment, the protective barrier modulates enzymatic activity, such as by enhancing the production or activation of enzymes that break down pathogens before they penetrate the epithelium.

The term "modulates enzymatic activity" refers to the enhancement or activation of enzymes that degrade or neutralize pathogens before they penetrate the epithelium, such as lysozyme or proteases. As a person skilled in the art would recognize, this can be determined by measuring the activity levels of key enzymes in the nasal environment after nanocapsule administration. An indicative threshold for enzymatic modulation would be an increase in enzymatic activity (e.g., lysozyme or protease activity) by at least 60% relative to untreated controls, with corresponding pathogen degradation rates improved by at least 70%.

In another preferred embodiment, the protective barrier is a hydration barrier, for instance, a moisture layer that traps and flushes out pathogens from the nasal passages.

The term "hydration barrier" refers to a moisture layer that traps pathogens and facilitates their removal from the nasal passages through mucociliary clearance. As a person skilled in the art would recognize, this can be assessed by monitoring nasal surface hydration levels and evaluating the clearance rate of fluorescently labeled particles designed to simulate pathogen movement. An indicative threshold for a hydration barrier would be an increase in nasal surface hydration by at least 30% from baseline, along with a clearance rate of fluorescent particles improved by at least 50% compared to untreated conditions.

Other embodiments of the first aspect of the invention are set below. Please note that all the embodiments of the first aspect of the invention apply herein:
- In a particular embodiment (hereinafter "particular embodiment #1") the nanocapsule system of the invention comprises or consists of: a) a surface layer comprising chitosan, b) an oily core, and c) a surfactant, the prophylactic treatment comprises or consists of preventing the respiratory pathogen from causing a symptomatic infection, and the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, **common** sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof.
   o In a particular embodiment, particular embodiment #1 further mentions: the oil consists of at least 90% w/w of triglycerides with three fatty acids having an aliphatic tail of 6 to 12 carbon atoms.
   o In a particular embodiment, particular embodiment #1 further mentions: the oil consists of at least 90% w/w of a mixture of caprylic (C8) and capric (C10) triglycerides.
   o In a particular embodiment, particular embodiment #1 further mentions: the oil consists of at least 90% w/w of a mixture of caprylic (C8) and capric (C10) triglycerides in a w/w ratio between 80:20 and 50:50
   o In a particular embodiment, particular embodiment #1 further mentions: the oil consists of at least 90% w/w of a mixture of caprylic (C8) and capric (C10) triglycerides in a w/w ratio between 75:25 and 65:35
   o In a particular embodiment, particular embodiment #1 further mentions: the oil consists of at least 90% w/w of a mixture of caprylic (C8) and capric (C10) triglycerides in a w/w ratio of 60:40.
   o In a particular embodiment, particular embodiment #1 further mentions: the oil consists of at least 90% w/w of Miglyol^{®} 812N.
   o In a particular embodiment, particular embodiment #1 further mentions: the oil consists of Miglyol^{®} 812N.
- In a particular embodiment (hereinafter "particular embodiment #2") the nanocapsule system of the invention comprises or consists of: a) a surface layer comprising chitosan, b) an oily core, and c) a surfactant, the prophylactic treatment comprises or consists of preventing the respiratory pathogen from causing a symptomatic infection, and the oily core consists of an oil selected from the list consisting of mineral oil, **squalene** oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, the surfactant is an oil-soluble surfactant selected from the list consisting of sorbitan esters, polyglycerol esters, glyceryl esters, propylene glycol esters, polyglyceryl fatty acid esters, sucrose esters, fatty acid esters, dioctyl sodium sulfosuccinate (AOT), polyoxyethylene sorbitol hexaoleate, lanolin alcohols and derivatives, fatty acid salts with hydrocarbon chains of 12 to 22 carbon atoms, and phospholipid-based compounds.
   o In a particular embodiment, particular embodiment #2 further mentions: the surfactant is a phospholipid-based compound selected from the list consisting of lecithin, phosphatidylglycerol, phosphatidylserine, phosphatidylinositol, diphosphatidylglycerol, phosphatidic acid, phosphatidylcholine and phosphatidylethanolamine.
   o **In a particular** embodiment, particular embodiment #2 further mentions: the surfactant is a phospholipid-based compound selected from the list consisting of Lipoid S75 (soy phospholipid with 70% phosphatidylcholine), hydrogenated lecithin, phosphatidylcholine, LIPOID S 45 (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%).
   o In a particular embodiment, particular embodiment #2 further mentions: the surfactant is Lipoid S75.
- In a particular embodiment (hereinafter "particular embodiment #3") the nanocapsule system of the invention comprises or consists of: a) a surface layer comprising chitosan, b) an oily core, and c) a surfactant, the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea **leaf oil ,** flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, and the oil consists of at least 90% w/w of Miglyol^{®} 812N and the surfactant is Lipoid S75.
   o In a particular embodiment, particular embodiment #3 further mentions: the prophylactic treatment comprises or consists of preventing the respiratory pathogen from causing a symptomatic infection.
   o In a particular embodiment, particular embodiment #3 further mentions: the prophylactic treatment comprises or consists of preventing the respiratory pathogen from causing a symptomatic infection, and the respiratory pathogen is a respiratory virus.
   o In a particular embodiment, particular embodiment #3 further mentions: the prophylactic treatment comprises or consists of preventing the respiratory pathogen from causing a symptomatic infection, and the respiratory pathogen is a respiratory virus selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses (e.g. HCoV-229E, HCoV-NL63, HCoV-OC43 and HCoV-HKU1), bocavirus, and enteroviruses (e.g. coxsackieviruses and echoviruses).
   o In a particular embodiment, particular embodiment #3 further mentions: the prophylactic treatment comprises or consists of preventing the respiratory pathogen from causing a symptomatic infection, and the respiratory pathogen is the influenza virus.
   o In a particular embodiment, particular embodiment #3 further mentions: the prophylactic treatment comprises or consists of preventing the respiratory **pathogen** from causing a symptomatic infection, and the respiratory pathogen is an influenza A virus.
   o In a particular embodiment, particular embodiment #3 further mentions: the prophylactic treatment comprises or consists of preventing the respiratory pathogen from causing a symptomatic infection, and the respiratory pathogen is the influenza virus.
- In a particular embodiment (hereinafter "particular embodiment #4") the nanocapsule system of the invention comprises or consists of: a) a surface layer comprising chitosan, b) an oily core, and c) a surfactant, the oily core consists of Miglyol^{®} 812N and the surfactant is Lipoid S75.
   o In a particular embodiment, particular embodiment **#4** further mentions: the prophylactic treatment comprises or consists of preventing the respiratory pathogen from causing a symptomatic infection.
   o In a particular embodiment, particular embodiment **#4** further mentions: the prophylactic treatment comprises or consists of preventing the respiratory pathogen from causing a symptomatic infection the respiratory pathogen is a respiratory virus.
   o In a particular embodiment, particular embodiment **#4** further mentions: the prophylactic treatment comprises or consists of preventing the respiratory pathogen from causing a symptomatic infection, and the respiratory pathogen is a respiratory virus selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses (e.g. HCoV-229E, HCoV-NL63, HCoV-OC43 and HCoV-HKU1), bocavirus, and enteroviruses (e.g. coxsackieviruses and echoviruses).
   o In a particular embodiment, particular embodiment **#4** further mentions: the prophylactic treatment comprises or consists of preventing the respiratory pathogen from causing a symptomatic infection, and the respiratory pathogen is the influenza virus.
   o In a particular embodiment, particular embodiment **#4** further mentions: the prophylactic treatment comprises or consists of preventing the respiratory pathogen from causing a symptomatic infection, and the respiratory pathogen is an influenza A virus.
   o In a particular embodiment, particular embodiment #4 further mentions: the prophylactic treatment comprises or consists of preventing the respiratory pathogen from causing a symptomatic infection, and the respiratory pathogen is the influenza virus.

A second aspect of the invention refers to a pharmaceutical composition comprising the nanocapsule system of the invention (as defined in the first aspect of the invention) and, optionally, one or more pharmaceutically acceptable excipients, for use in the prophylactic treatment of an infection caused by a respiratory pathogen in a subject in need thereof, wherein the nanocapsule system is administered to the subject intranasally.

The nanocapsule system and the prophylactic treatment for the prophylactic treatment of an infection caused by a respiratory pathogen are the same as for the first aspects of the invention. Thus, all the embodiments mentioned in this regard for the first aspect apply herein.

The term "pharmaceutical composition" refers to a formulation that combines the nanocapsule system of the invention with other excipients or inactive substances to create a product intended for medical use. These excipients may include fillers, binders, stabilizers, preservatives, solvents, and other agents that help in the delivery, stability, and absorption of the nanocapsule or its components in the body. Pharmaceutical compositions can be in various forms, such as tablets, capsules, liquids, creams, or injectable solutions, depending on the intended route of administration and therapeutic purpose. In the context of the present invention the pharmaceutical composition may be specifically designed to be suitable for intranasal administration, ensuring proper delivery, absorption, and stability when applied to the nasal mucosa.

The term "pharmaceutically acceptable excipient" refers to a pharmaceutically acceptable excipient is a substance added to a drug formulation alongside the main medicinal component (in this case the nanocapsule system of the invention) to serve various purposes in the medicine's composition. These substances are typically inert and carefully evaluated for safety. They may aid in manufacturing, improve stability or bioavailability, enhance patient acceptability, or facilitate drug delivery. Excipients can include binders, disintegrants, fillers, preservatives, and lubricants, among others. While generally considered inactive, some excipients may have known effects in certain circumstances. In more complex drug delivery systems, such as nanocapsules, the distinction between excipients and active ingredients can become blurred, as all components of the nanoparticle may contribute to the efficacy and safety profiles of the final drug product.

The present invention also refers to the use of the nanocapsule system as defined in the first aspect of the invention for manufacturing a pharmaceutical composition for the prophylactic treatment of an infection caused by a respiratory pathogen in a subject in need. The "nanocapsule system", "prophylactic treatment" and "infection caused by a respiratory pathogen" are the same as for the first aspect of the invention. Thus, all the embodiments mentioned in these regards for the first aspect apply herein.

### EXAMPLES

The present invention is illustrated by means of the Examples set below without the intention of limiting its scope of protection.

### Example 1. Materials and methods

### 1.1. Preparation of solutions

Notes: let all components reach RT before use (approximately 20 minutes).

### 1.1. 1. Solution of Chitosan HCl in ultrapure water (0.25 mg/mL)

Weight 5 mg of Chitosan HCl in a disposable sterile ultra-clear polypropylene container (50 mL), equip with a stirring magnet, measure 20 mL of ultrapure water using the 25 mL graduated cylinder and add them, cap the container, and stir at RT for at least 60 minutes using the multipoint magnetic stirrer. Use within the preparation day.

Once perfectly dissolved, 10 mL of chitosan solution should be filtered through a 0.22 µm filter. The solution is aspirated employing a needle and a 10 mL syringe. Then, it is pushed out across the filter. The filtered solution should be kept in a new disposable sterile ultra-clear polypropylene container.

### 1.1.2. Solution of Lipoid S75 in ethanol (40 mg/mL)

Weight 40 mg of Lipoid S75 in a 10 mL glass vial (ideally transparent), equip with a stirring magnet, add 1 mL of ethanol using an automatic pipette, cap the vial, and stir at RT for at least 30 minutes using the multipoint magnetic stirrer. If needed, vortex the mixture to ensure complete homogenization. Use within the preparation day.

### 1.2. Emulsification

### 1.2.1. Preparation of the aqueous phase (2.7 mL)

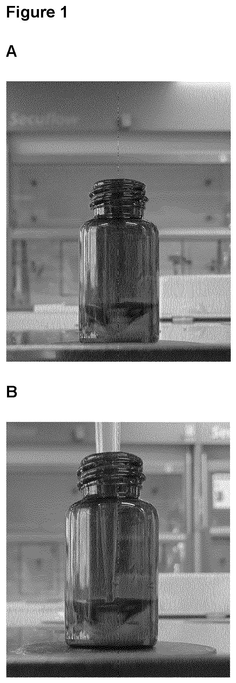

Measure 2.7 mL of the chitosan solution using the 1000-5000 µL automatic pipette, pour them onto a 10 mL amber glass vial equipped with a stirring magnet, and stir at 700 rpm or until a stable vortex is achieved, using the multipoint magnetic stirrer (Figure 1A).

### 1.2.2. Preparation of the organic phase 2x (0.678 mL)

**In a 1.5 mL amber Eppendorf tube, add 300 µL of Lipoid S75 solution (40 mg/mL), 78 µL of Miglyol 812N and 300 µL of ethanol. Close the cap. Vortex briefly. Use immediately.**

### 1.2.3. Preparation of the emulsion (3.039 mL)

**Pour 339 µL of the organic phase at once over the center of the vortex of the aqueous phase using a 100-1000 µL automatic pipette, as closest to the bottom as possible without touching the aqueous solution with the pipette tip (****Figure 1B****).**

**Mixture turns white immediately. Cap and stir for 5 minutes, then let rest for another 5 minutes.**

### Example 2. Formulations with other chitosans

**In the initial phase of formulation development, the use of different chitosans was studied (Table 2). We finally selected chitosan HCl (HMC) for the subsequent phases of qualitative and quantitative composition optimization. The exact composition of the chitosan nanocapsules (hereinafter CS nanocapsules) is shown in Table 3.**

**Table 2. Characteristics of formulations containing chitosan as a polymer.**

| **Formulations Screening** | | | | | | | |
|---|---|---|---|---|---|---|---|
| **Polymer** | **Solvent** | **Solvent %** | **[Polymer]** | **Size (nm)** | **Pdl** | **Zpot (mV)** | **pH** |
| CS HCl | H2O | | 1mg/mL | 168,3 | 0,184 | 39 | 4,49 |
| CS Ultrapure | H2O | | 1mg/mL | 341 | 0,445 | 5,56 | - |
| AL-120 - 80% DDA | HCl 1N | 0,75 | 1mg/mL | 1,59E+04 | 0,195 | 66,2 | 3,11 |
| | | | 0,5mg/mL | 1,92E+04 | 1 | 53,3 | 3,25 |
| | | 0,92 | 1mg/mL | 994,9 | 1 | 64 | 2,5 |
| | Acetic Acid | 1 | 1mg/mL | 1,29E+04 | 0,049 | 72 | 2,73 |
| | | | 0,5mg/mL | 1,31E+04 | 0,345 | 69,8 | 2,83 |
| AL-476 - 90% DDA | HCl 1N | 0,75 | 1mg/mL | 4582 | 0,279 | 62,3 | 1,96 |
| | | | 0,5mg/mL | 1771* | 1 | 55,2 | 2,26 |
| | | 0,89 | 1mg/mL | 1,03E+04 | 1 | 64,5 | 2,35 |
| | Acetic Acid | 1 | 1mg/mL | 7308 | 0,51 | 72,2 | 2,81 |
| | | | 0,5mg/mL | 1,28E+04 | 0,458 | 64,1 | 2,84 |
| A-90101 - 90,1% DDA | HCl 1N | 0,5 | 1mg/mL | 2247 | 1 | 45,8 | 3,32 |
| | | | 0,5mg/mL | 2232 | 1 | 41,9 | 3,41 |
| | | 0,75 | 1mg/mL | 309,4 | 0,198 | 54,6 | 2,73 |
| | | | 0,5mg/mL | 1776 | 1 | 34,9 | 2,58 |
| | Acetic Acid | 1 | 1mg/mL | 1922 | 1 | 72,2 | 2,8 |
| | | | 0,5mg/mL | 3,46E+04 | 0,478 | 64,1 | 2,92 |
| CM95401132 - 98% DDA | HCl 1N | 0,5 | 1mg/mL | 771,3 | 1 | 5,01 | 3,37 |
| | | | 0,5mg/mL | 6,52E+04 | 1 | 19,3 | 3,55 |
| | | 0,61 | 1mg/mL | 4,25E+04 | 1 | 70,5 | 3,05 |
| | Acetic Acid | 1 | 1mg/mL | 3,46E+04 | 1 | 77,9 | 2,74 |
| | | | 0,5mg/mL | 7,34E+04 | 1 | 75,6 | 2,85 |

**Table 3. Composition of the chitosan nanocapsules (CS nanocapsules)**

| **Product** | **%(w/w)** |
|---|---|
| Miglyol 812N | 1.24 |
| Ethanol 100% | 7.49 |
| Lipoid S75 | 0.2 |
| CS HCl GMP | 0.02 |
| Water | 90.59 |

### Example 3. Comparison of in vitro toxicities with other formulations without chitosan

In later phases of the project, three nanoformulations were studied, all with similar properties and quality attributes but using different polymers. The following figures show the qualitative composition of each of them **(Table 4),** as well as the values of their quality attributes **(Table 5),** all of which are very similar regardless of the polymer used.

**Table 4. Qualitative compositions of the different formulations studied.**

| **Chitosan formulation** | **Polyarginine formulation** | **Protamine formulation** |
|---|---|---|
| Miglyol 812N | Miglyol 812N | DL-α-tocopherol |
| Ethanol 100% | Ethanol 100% | Tween 80 |
| Lipoid S75 | Lipoid S75 | Sodium cholate |
| CS HCl | nBu-PArg | Ethanol 100% |
| MilliQ water | MilliQ water | Protamine YGK |
| | | MilliQ water |

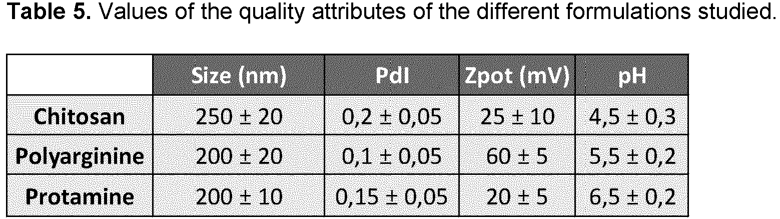

The toxicities of the formulations were determined in RAW 264.7 cells (mouse macrophages), previously stimulated with LPS (lipopolysaccharide), which allows monocytes to differentiate into a proinflammatory state (as M1 macrophages). Different dilutions of the three formulations were studied over a 24-hour incubation period, allowing the generation of dose-dependent cell viability curves and their TC50 value, which corresponds to the concentration at which a formulation causes 50% cell death. Comparing the results, a clear difference in toxicity between them is observed. The chitosan prototype (hereinafter CS nanocapsules) - which comprises 0.225 mg/ml of chitosan - is clearly less toxic, not reaching 50% toxicity until a 1:4 dilution (1 part of the initial formulation diluted in 3 parts of cell medium). Protamine and polyarginine have more similar toxicities, although it is true that polyarginine is somewhat more toxic, especially because from 1:32 cell viability declines much faster **(****Figure 2****).**

### Example 4. Comparison of in vitro efficacy with other chitosan-free formulations

After completing the toxicity study, the efficacy of LPS treatment in reducing inflammation in macrophages was studied. To this end, the cytokine levels present in the supernatants from previous toxicity experiments were analyzed using ELISA. Specifically, the study of the levels of the proinflammatory cytokine IL-6 showed a significant reduction (p=0.030), proving the anti-inflammatory capacity of the chitosan formulation. It was the only one that managed to reduce these values with a significance of ** **(****Figure 3****).**

### Example 5. Animal studies

The results of the influenza efficacy experiments in animals, with intranasal treatments of 5µL per nostril of the nanoformulation described in **Table 3** on days -1, +1 and +3 (0 being the day of infection with H1N1 PR8 influenza) **(****Figure 4****),** were performed in duplicate to ensure reproducibility and showed an unprecedented capacity to prevent influenza contagion. Thus, 100% of the animals treated with our CS nanocapsules formulation did not develop infection after having been in contact with the virus. This fact was fully validated by analyzing multiple parameters such as their weight **(****Figure 5****),** levels of inflammation in the lung **(****Figure 6****),** levels of inflammation in the blood **(****Figure 7****)** and viral load in the lung **(****Figure 8****),** all of which corresponded to values typically found in non-infected animals.

### Example 6. In vitro toxicity of the chitosan nanocapsules in 293T cells

A cellular cytotoxicity assessment by cell metabolic activity was performed in 293-ACE2 cells in order to determine the maximum amount of nanoformulation that can be employed in viral entry studies. The experiment was performed 3 times in triplicates for 8 dilutions of the nanocapsules (NPs). 293-ACE2 cells were incubated with different dilutions of the formulation in cell media for 48h. Then, cells were washed with PBS and ATP-dependent light emission was measured as an indication of cell viability.

The results showed a dose-dependent toxicity. Moreover, it should be noted that even the highest volume of formulation studied (100 µL of formulation, which is the maximum for the experimental design of viral entry studies, and which is equivalent to a 1:2 dilution of the formulation shown in **Table 3),** is not toxic for cells, as it does not reduce cell viability more than 10-15%.

### Example 7. Viral entry study to search for pan-coronavirus entry inhibition of NPs in ACE-2 expressing 293T cells

In this study, the inhibition of SARS-CoV-2 pseudovirus fusion was studied in 293-ACE2 cells using the nanoformulation (NPs) described in **Table 3.** The assay is based on a single-cycle, envelope-deficient HIV-1 reporter virus expressing the luciferase gene. This pseudovirus is pseudotyped with the SARS-CoV-2 spike protein, which facilitates viral fusion with 293-ACE2 cells. The pseudovirus was combined with the formulation at different concentrations and the mixture was incubated with the cells for 48h to detect viral entry. Luciferase expression served as a readout for successful fusion events.

The results showed a clear dose-dependent inhibitory effect of the tested NPs on viral fusion. The formulation effectively reduces viral fusion at concentrations that do not exhibit cytotoxicity in 293-ACE2 cells (as demonstrated in **Example 6** and **Figure 9****).** Moreover, we can see that the formulation at higher concentrations is able to inhibit fusion as effectively as a recombinant murine Fc-ACE2 protein or an HIV-1 integration inhibitor such as dolutegravir.

## Claims

1. A nanocapsule system, which comprises:
a) a surface layer comprising chitosan,
b) an oily core, and
c) a surfactant,
for use in the prophylactic treatment of an infection caused by a respiratory pathogen in a subject in need thereof, wherein the nanocapsule system is administered to the subject intranasally.

2. The nanocapsule system according to claim 1, wherein the prophylactic treatment comprises preventing the respiratory pathogen from causing a symptomatic infection.

3. The nanocapsule system according to any of the claims 1 or 2, for use in the prophylactic treatment of an infection caused by a respiratory virus.

4. The nanocapsule system according to claim 3, wherein the respiratory virus is selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses, bocavirus, and enteroviruses.

5. The nanocapsule system according to claim 4, wherein the respiratory virus is the influenza virus.

6. The nanocapsule system according to any of the claims 1 to 5, wherein the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof.

7. The nanocapsule system according to any of the claims 1 to 6, wherein the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, and wherein the prophylactic treatment comprises preventing the respiratory pathogen from causing a symptomatic infection.

8. The nanocapsule system according to any of the claims 6 or 7, wherein the oil consists of at least 90% w/w of triglycerides with three fatty acids having an aliphatic tail of 6 to 12 carbon atoms.

9. The nanocapsule system according to any of the claims 6 to 8, wherein the oil consists of at least 90% w/w of a mixture of caprylic (C8) and capric (C10) triglycerides.

10. The nanocapsule system according to any of the claims 6 to 9, wherein the oil consists of at least 90% w/w of a mixture of caprylic (C8) and capric (C10) triglycerides in a w/w ratio between 80:20 and 50:50.

11. The nanocapsule system according to any of the claims 1 to 10, wherein the surfactant is an oil-soluble surfactant.

12. The nanocapsule system according to claim 11, the oil-soluble surfactant is a phospholipid-based compound selected from the list consisting of lecithin, phosphatidylglycerol, phosphatidylserine, phosphatidylinositol, diphosphatidylglycerol, phosphatidic acid, phosphatidylcholine and phosphatidylethanolamine.

13. The nanocapsule system according to claim 12, wherein the phospholipid-based compound is selected from the list consisting of Lipoid S75 (soy phospholipid with 70% phosphatidylcholine), hydrogenated lecithin, phosphatidylcholine, LIPOID S 45 (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%).

14. The nanocapsule system according to claim 13, wherein the phospholipid-based compound is Lipoid S75 (soy phospholipid with 70% phosphatidylcholine).

15. The nanocapsule system according to any of the claims 1 to 14, wherein the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof, wherein the oil consists of at least 90% w/w of a mixture of caprylic (C8) and capric (C10) triglycerides in a w/w ratio between 80:20 and 50:50, wherein the surfactant is Lipoid S75 (soy phospholipid with 70% phosphatidylcholine), for use in the prophylactic treatment of an infection caused by the influenza virus.

## Amended claims

### Amended claims in accordance with Rule 137(2) EPC.

1. A nanocapsule system, which comprises:
a) a surface layer consisting of chitosan,
b) an oily core, and
c) a surfactant,
for use in the prophylactic treatment of an infection caused by a respiratory virus in a subject in need thereof, wherein the nanocapsule system is administered to the subject intranasally.

2. The nanocapsule system according to claim 1, wherein the respiratory virus is selected from the group consisting of influenza virus, respiratory syncytial virus (RSV), human rhinovirus (HRV), human parainfluenza virus (HPIV), adenovirus, human metapneumovirus (hMPV), severe acute respiratory syndrome coronavirus 2 (SARS-CoV-2), severe acute respiratory syndrome coronavirus (SARS-CoV), Middle East respiratory syndrome coronavirus (MERS-CoV), endemic human coronaviruses, bocavirus, and enteroviruses.

3. The nanocapsule system according to claim 2, wherein the respiratory virus is the influenza virus.

4. The nanocapsule system according to claim 2, wherein the respiratory virus is the severe acute respiratory syndrome coronavirus 2 (SARS-CoV-2).

5. The nanocapsule system according to claim 1, wherein the nanocapsule system consists of:
a) a surface layer consisting of chitosan,
b) an oily core, and
c) a surfactant.

6. The nanocapsule system according to claim 5, wherein the respiratory virus is the influenza virus.

7. The nanocapsule system according to claim 5, wherein the respiratory virus is the respiratory virus is the severe acute respiratory syndrome coronavirus 2 (SARS-CoV-2).

8. The nanocapsule system according to any of the claims 5 to 7, wherein the oily core consists of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil, flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof.

9. The nanocapsule system according to claim 8, wherein the oil is a medium-chain triglyceride having at least 90% w/w of triglycerides with three fatty acids having an aliphatic tail of 6 to 12 carbon atoms.

10. The nanocapsule system according to claim 9, wherein the oil is a medium-chain triglyceride having at least 90% w/w of a mixture of caprylic (C8) and capric (C10) triglycerides.

11. The nanocapsule system according to any of the claims 1 to 10, wherein the surfactant is an oil-soluble surfactant.

12. The nanocapsule system according to claim 11, the oil-soluble surfactant is a phospholipid-based compound selected from the list consisting of lecithin, phosphatidylglycerol, phosphatidylserine, phosphatidylinositol, diphosphatidylglycerol, phosphatidic acid, phosphatidylcholine and phosphatidylethanolamine.

13. The nanocapsule system according to claim 12, wherein the phospholipid-based compound is selected from the list consisting of Lipoid S75 (soy phospholipid with 70% phosphatidylcholine), hydrogenated lecithin, phosphatidylcholine, LIPOID S 45 (lecithin fraction with phosphatidylcholine content ≥ 45%), PHOSPHOLIPON^{®} 90 G (phosphatidylcholine content ≥ 94%), LIPOID P 75 (phospholipids with phosphatidylcholine content ≥ 70%), LIPOID P 100 (phosphatidylcholine content ≥ 90%), LIPOID H 20 (lecithin fraction with phosphatidylcholine content ≥ 20%), LIPOID H 100 (phosphatidylcholine content ≥ 94%), PHOSPHOLIPON^{®} 80 H (hydrogenated phospholipids with phosphatidylcholine content ≥ 70%), PHOSPHOLIPON^{®} 90 H (hydrogenated phosphatidylcholine content ≥ 90%), LIPOID P 75-3 (hydrogenated phospholipids with phosphatidylcholine content ≥ 62%), LIPOID P 100-3 (hydrogenated phosphatidylcholine content ≥ 90%), and LIPOID H 100-3 (hydrogenated phosphatidylcholine content ≥ 90%).

14. The nanocapsule system according to claim 6, wherein the respiratory virus is the influenza virus and the nanocapsule system consists of:
a) a surface layer consisting of chitosan;
b) an oily core consisting of an oil selected from the list consisting of mineral oil, squalene oil, flavor oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicarpate ketals, decyl oleate, C12-C1S alkyl lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petrolatum, argan oil, rapeseed oil, chili oil, coconut oil, fractionated coconut oil, corn oil, cottonseed oil, flax oil, oil of grape seed, mustard oil, olive oil, palm oil, fractionated palm oil, peanut oil, oil castor oil, pine seed oil, poppy seed oil, pumpkin seed oil, rice bran oil, safflower, tea oil, truffle oil, vegetable oil, apricot oil, jojoba oil, macadamia oil, wheat germ oil, almond oil, soybean oil, sesame oil, hazelnut oil, sunflower oil, hemp oil, bois oil, Kukui nut oil, avocado oil, walnut oil, fish oil, berry oil, allspice oil, juniper oil, seed oil, almond seed oil, anise seed oil, celery seed oil, cumin seed oil, seed oil nutmeg, basil leaf oil, bay leaf oil, cinnamon leaf oil, common sage leaf oil, eucalyptus leaf oil, lemon leaf oil, melaleuca leaf oil, oregano oil, patchouli oil, peppermint oil, pine needle oil, rosemary leaf oil, spearmint oil, tea tree leaf oil, thyme leaf oil, Canada tea leaf oil , flower oil, chamomile oil, roman sage oil, clove oil, ac geranium flower eite, hyssop flower oil, jasmine flower oil, lavender flower oil, mauka flower oil, marjoram flower oil, orange flower oil, rose flower oil, oil ylang-ylang flower, bark oil, cassia bark oil, cinnamon bark oil, sassafras bark oil, wood oil, camphor wood oil, cedar wood oil, rosewood oil, sandalwood oil, gingerwood oil, resin oil, recin oil, myrrh oil, skin oil, Bergamo skin oil, grapefruit skin oil, lemon skin oil, lime skin oil, orange peel oil, tangerine skin oil, root oil, valerian oil, oleic acid, linoleic acid, oleyl alcohol, isostearyl alcohol, ethyl oleate, long-chain monoglycerides, long-chain diglycerides, long-chain triglycerides, medium-chain monoglycerides, medium-chain diglycerides, medium-chain triglycerides, synthetic or semi-synthetic derivatives and combinations thereof; and
c) a surfactant, wherein the surfactant is an oil-soluble surfactant.

15. The nanocapsule system according to claim 7, wherein the respiratory virus is the severe acute respiratory syndrome coronavirus 2 (SARS-CoV-2); and the nanocapsule system is as defined in claim 14.
